# FASCICULE DE BREVET EUROPEEN

(11) **EP 1 234 019 B1**
(45) Date de publication et mention de la délivrance du brevet: **11.02.2009**
(21) Numéro de dépôt: 00979728.3
(22) Date de dépôt: 14.11.2000
(51) Int. Cl.: C12N 1/04

(54) **PROCEDE DE SECHAGE DES BACTERIES**
VERFAHREN ZUM TROCKNEN VON MIKROORGANISMEN
METHOD FOR DRYING BACTERIA

(30) Priorité: 18.11.1999 FR 9914507
(43) Date de publication de la demande: 28.08.2002
(73) Titulaire: DANISCO A/S, 1411 Copenhagen K. (DK)
(72) Inventeur: DIGUET, Sylvain, CH-8595 Kreuzlingen (CH)
(74) Mandataire: Touati, Catherine
(86) Numéro de dépôt international: PCT/FR2000/003164
(87) Numéro de publication internationale: WO 2001/036590

(56) Documents cités:
- EP-A- 0 818 529
- EP-A- 0 906 951
- WO-A-98/10666
- FR-A- 2 290 846
- US-A- 4 423 079
- DATABASE WPI Section Ch, Week 198745 Derwent Publications Ltd., London, GB; Class D13, AN 1987-319054 XP002145298 & SU 1 292 706 A (APPL BIOCHEM RES), 28 février 1987 (1987-02-28)
- DATABASE WPI Section Ch, Week 198648 Derwent Publications Ltd., London, GB; Class D13, AN 1986-318102 XP002145299 & SU 1 227 145 A (AS UKR HEAT PHYS), 30 avril 1986 (1986-04-30)

## Description

La présente invention a pour objet un procédé de séchage de bactéries mettant en oeuvre deux étapes successives : une première étape de pulvérisation dans une atmosphère chaude appelée également atomisation suivie d'une deuxième étape de séchage sous pression réduite.

Les bactéries en général et les bactéries lactiques en particulier sont des organismes vivants qui, en présence d'eau, évoluent dans le temps. L'utilisation de ces produits de manière différée impose une conservation de l'activité bactérienne dans le temps qui ne peut être obtenue que par arrêt du métabolisme des bactéries. L'une des voies pour arrêter le métabolisme des bactéries est de les mettre sous forme sèche. En effet, à l'état sec, les bactéries présentent une plus grande stabilité qu'en suspensions aqueuses.

La transformation d'une suspension de bactérie à l'état sec est une opération délicate. Afin de maintenir le potentiel de viabilité des bactéries tout au long du procédé de séchage, il est important de bien maîtriser les différentes contraintes (thermique, mécanique, chimique) auxquelles ces bactéries sont soumises.

Dans la pratique, le séchage des bactéries est toujours effectué par lyophilisation en présence d'une quantité importante d'agents de support ou de dessiccation, qui ont un rôle crucial. On peut décrire la lyophilisation comme un procédé en deux étapes : une première étape consistant à congeler les bactéries dans une matrice amorphe contenant beaucoup d'eau, et une deuxième étape consistant en la sublimation de l'eau de la matrice sous une pression fortement réduite.

A l'heure actuelle, la lyophilisation est le procédé qui permet de conserver le mieux l'activité bactérienne lors du séchage. Cependant, la lyophilisation présente certains inconvénients : sa productivité faible (les cinétiques de sublimation sont très lentes) et son coût (production du froid nécessaire à la congélation et de la pression fortement réduite nécessaire à la sublimation).

Ainsi, de nombreuses tentatives ont été faites pour substituer la lyophilisation par un procédé de séchage plus productif et moins coûteux. Le séchage par atomisation a été de loin le procédé le plus étudié. Les taux de survie des bactéries après atomisation dépendent de la nature de la bactérie. Ces taux atteignent rarement 60 % des taux obtenus avec la lyophilisation.

Un autre procédé de séchage largement étudié est le séchage par lit fluidisé, qui est en fait constitué de particules solides fluidisées servant de support. Dans ce procédé, la suspension de bactéries à sécher est pulvérisée sur le support fluidisé. Bien que les taux de survie des bactéries par ce procédé soient supérieurs à ceux obtenus par atomisation (taux inférieurs à 70 % par rapport à ceux obtenus par lyophilisation), ce procédé demeure néanmoins moins performant que la lyophilisation. De plus, la mise en oeuvre d'un support dans ce type de procédé n'est pas toujours compatible avec l'application finale de la bactérie séchée.

Quel que soit le procédé de séchage, un paramètre important à prendre en compte est la nature et la quantité d'agents protecteurs préservant les bactéries des pertes d'activités dues à la dessicative. Ces agents sont en général mélangés avec les bactéries pour les stabiliser et pour empêcher toute perte d'activité de ces dernières lors du séchage.

Aujourd'hui, il apparaît qu'il n'existe aucun procédé de séchage qui puisse être présenté comme une alternative à la lyophilisation, c'est-à-dire un procédé qui présente les avantages de la lyophilisation en particulier un taux de survie comparable sans ses inconvénients notamment en termes de productivité relativement faible et de coût élevé.

Le but de la présente invention est en particulier de résoudre ce problème.

Un autre but de la présente invention est de proposer un procédé de séchage tel que défini plus haut, qui soit efficace et compatible avec tout type de bactéries.

L'invention a encore pour but de proposer des bactéries sous forme sèche, qui puissent être stockées à des températures proches de l'ambiante (environ 20°C) et qui retrouvent leur activité lors de leur réhydratation en milieux aqueux.

D'autres avantages et caractéristiques de la présente invention apparaîtront clairement à la lecture de la description et les exemples qui vont suivre.

Dans le cadre de la présente invention, le terme « bactérie (s) » désignera à la fois des organismes eucaryotes et procaryotes.

Dans le cadre de la présente invention, le terme "formulation bactérienne" désigne plus particulièrement une biomasse dans laquelle une ou plusieurs bactérie(s) au sens de la présente invention, appartenant au même genre ou à des genres différents se trouve(nt) en mélange avec des agents de protection et éventuellement d'autres additifs usuels employés lors du séchage des bactéries.

La présente invention a donc pour objet un procédé de séchage de formulation bactérienne mettant en oeuvre deux étapes successives : une première étape de pulvérisation dans une atmosphère chaude appelée également atomisation suivie d'une deuxième étape de séchage sous pression réduite.

Ainsi, la présente invention a pour objet un procédé de séchage de formulations bactériennes caractérisé en ce que :
*a)* l'on pulvérise une suspension aqueuse d'au moins une bactérie représentant au moins 3% de la matière sèche de la suspension, dans une atmosphère chaude (atomisation) dont la température est d'au plus 500°C pendant un temps adapté pour obtenir une poudre dont la teneur résiduelle en eau est d'au moins 5 % en poids par rapport au poids total de la poudre, et
*b)* l'on soumet la poudre issue de l'étape *(a)* à une étape de séchage sous pression réduite d'au plus 3.10⁴ Pa pour obtenir une poudre dont la teneur résiduelle en eau est d'au plus 4 % en poids par rapport au poids total de la poudre.

Le procédé de l'invention peut être appliqué à tout type de bactéries et de champignons.

A titre de bactéries, on peut citer par exemple les bactéries appartenant avantageusement aux genres *Lactobacillus, Streptococcus, Leuconostoc, Lactococcus, Pediococcus, Staphylococcus, Bifidobacterium, Camobacterium, Enterococcus, Propionibacterium, Brevibacterium, Corynebacterium, Arthrobacter,* ou *Hafnia*, et de préférence aux espèces *Lactobacillus brevis, Lactobacillus plantarum, Lactobacillus casei, Lactobacillus acidophilus, Lactobacillus fermentum, Lactobacillus rhamnosus, Lactobacillus paracasei, Lactobacillus johnsonii, Lactobacillus sakei, Lactobacillus delbrueckii, Lactobacillus helveticus, Lactobacillus curvatus, Streptococcus salivarius, Streptococcus infantarius, Streptococcus thermophilus, Lactococcus lactis, Leuconoctoc mesenteroides, Oenococcus oeni, Pediococcus pentosaceus, Pediococcus acidilactici, Staphylococcus camosus, Staphylococcus xylosus, Bifidobacterium lactis, Bifidobacterium animalis, Bifidobacterium longum, Bifidobacterium bifidum, Enterococcus faecium, Brevibacterium linens, Corynebacterium flavescens, Arthrobacter nicotianae, Hafnia alvei*.

A titre de champignons, on peut citer par exemple les champignons appartenant avantageusement aux genres, *Penicillium, Candida, Geotrichum, Kluyveromyces, Rhodosporidium* ou *Debaryomyces*, et de préférence aux espèces, *Debaryomyces hansenii, Candida famata, Candida utilis, Geotrichum candidum, Kluyveromyces lactis, Rhodosporidium infirmominiatum, Trichothecium domesticum, Penicillium candidum*.

Dans un premier temps, on prépare la suspension bactérienne. Ladite suspension peut être préparée par un procédé de fermentation classique connu de l'homme du métier. On peut par exemple réaliser une fermentation discontinue éventuellement suivie par au moins une étape de concentration par des moyens habituels comme la centrifugation ou l'ultrafiltration.

Des agents de protection adaptés au séchage par atomisation notamment :
- des composés polyhydroxylés comme par exemple la cellulose éventuellement modifiée, le lactose, le saccharose, le tréhalose, le galactose, l'amidon, les hydrolysats d'amidon, les galactomannanes éventuellement modifiés, les carraghénanes, les pectines, les dextrines, le sorbitol ;
- des protéines comme par exemple les protéines dérivées du lait comme la caséine, la β-lactoglobuline, l'α-lactalbumine, le sérum albumine, les protéines dérivées de soja, les albumines, les globulines, les glutélines, les prolamines, les histones, les protamines ;
- des acides aminés comme par exemple la lysine, la cystéine, le glycine, le glutamate de sodium ;
- des vitamines ;
seuls ou en mélanges, et éventuellement d'autres additifs usuels employés lors du séchage des formulations bactériennes, sont alors ajoutés à la suspension éventuellement concentrée.

A titre d'agent de protection, on peut également utilisé le lait et ses dérivés, comme par exemple le lactosérum, le perméat de lactosérum, seuls ou en mélanges avec les agents précités.

La teneur en bactéries dans la suspension est comprise entre 10 et 400, avantageusement 10 et 200, de préférence entre 50 et 200, et plus préférentiellement entre 80 et 150, grammes de bactéries par kilogrammes de suspension.

Les additifs précités sont ajoutés en une quantité telle que le rapport bactérie(s)/additifs soit compris entre 0,25 et 9, et de préférence entre 0,3 à 2.

La suspension, avant l'étape de séchage par atomisation, est avantageusement maintenue à une température comprise entre 0 et 50°C, et préférentiellement entre 2 et 20°C.

La pulvérisation de la suspension bactérienne dans une atmosphère chaude (spray-drying) appelée également atomisation, peut être réalisée au moyen de tout pulvérisateur connu en soi, par exemple par une buse de pulvérisation du type pomme d'arrosoir ou autre. On peut également utiliser des atomiseurs dits à turbine. Sur les diverses techniques de pulvérisation susceptibles d'être mise en oeuvre dans le présent procédé, on pourra se référer notamment à l'ouvrage de base de K. MASTERS intitule "SPRAY-DRYING HANDBOOK" (quatrième édition, 1985, Editions John Wiley & Sons, Inc.- New York).

On notera que l'on peut également mettre en oeuvre l'opération d'atomisation-séchage au moyen d'un réacteur "flash", par exemple du type mis au point par la Demanderesse et décrit notamment dans les demandes de brevet français numéros 2 257 326, 2 419 754 et 2 431 321. Dans ce cas, les gaz traitants (gaz chauds) sont animés d'un mouvement hélicoïdal et s'écoulent dans un puits-tourbillon. La suspension à sécher est injectée suivant une trajectoire confondue avec l'axe de symétrie des trajectoires hélicoïdales desdits gaz, ce qui permet de transférer parfaitement la quantité de mouvement des gaz au mélange à traiter. Les gaz assurent ainsi en fait une double fonction : d'une part la pulvérisation, c'est à dire la transformation en fines gouttelettes, de la suspension initiale, et d'autre part le séchage des gouttelettes obtenues. Par ailleurs, le temps de séjour extrêmement faible (généralement inférieur à 1/10 de seconde environ) des particules dans le réacteur présente pour avantage, entre autre, de limiter d'improbables risques de surchauffe par suite d'un contact trop long avec les gaz chauds.

L'air ou l'azote (concentration supérieure à 95%) peuvent être utilisés comme gaz chauds.

La température de l'atmosphère de séchage peut varier dans de larges limites, et elle dépend notamment du mode de mise en contact des gaz chauds et de la suspension à sécher, de la géométrie de la tour d'atomisation, ainsi que du temps de séjour moyen que l'on désire ou que l'on peut imposer au produit atomisé une fois dans ladite atmosphère. Dans le cadre de la présente invention, les conditions de l'atomisation (températures et/ou temps du séjour) sont déterminées de manière à n'éliminer que l'eau faiblement liée aux bactéries afin de limiter leur stress.

Dans la présente invention, le temps de séjour et/ou les températures sont déterminés de manière à obtenir une poudre de formulation bactérienne possédant une teneur résiduelle en eau comprise entre 5 et 30% en poids, et préférentiellement entre 7 et 20 % en poids par rapport au poids total de la poudre.

L'atomisation est effectuée de préférence à une température des gaz chauds à l'entrée d'au plus 500°C, avantageusement entre 70 et 300°C, et plus préférentiellement entre 70°C et 150°C.

Lorsque l'atomisation est bien contrôlée, elle permet de figer rapidement la matrice sous forme amorphe diminuant la destruction des bactéries par déchirement ou éclatement des membranes par contrainte mécanique (sous l'effet osmotique et/ou la croissance cristalline).

Il est préférable qu'à l'issue de l'étape *(a)* la poudre ait une granulométrie comprise entre 1 et 100 microns, et préférentiellement entre 5 et 50 microns. La granulométrie a été déterminée par un granulomètre laser de type MALVERN.

A l'issue de l'étape *(a)*, la formulation bactérienne est sous forme d'une poudre dont l'activité bactérienne a été préservée et dont la teneur résiduelle en eau ne permet pas d'assurer une stabilité suffisante de l'activité bactérienne au stockage à des températures supérieures à 4°C.

Le procédé de séchage est accompli par une étape ultérieure de séchage sous pression réduite *(b)*.

Dans cette étape, le séchage a lieu sous une pression réduite comprise avantageusement entre 1.10³ et 1,5.10⁴ Pa, de préférence entre 1,5.10³ et 1.10⁴ Pa.

Les températures utilisées sont comprises entre 20 et 60°C, de préférence entre 30 et 50°C.

Ce mode de séchage est particulièrement compatible avec les bactéries au sens de la présente invention et permet, lorsque l'on souhaite atteindre une très faible quantité d'eau résiduelle, un séchage de plusieurs heures sans diminution de l'activité bactérienne.

La poudre finale possède une teneur résiduelle en eau comprise entre 1 et 4% en poids, et préférentiellement entre 1 et 3 % en poids par rapport au poids total de la poudre.

Le séchage sous vide peut être réalisé par exemple avec un mélangeur-sécheur conductif sous vide de type DRAIS ou avec une sphère sous vide de type GLATT.

L'avantage du procédé selon la présente invention réside principalement dans le fait que l'association des deux étapes de séchage telles que décrites plus haut permet d'atteindre un taux de survie des bactéries supérieur à 50 % du taux obtenu avec la lyophilisation et le plus souvent supérieur à 70%, voire supérieur à 100%.

Un autre avantage de l'invention est le fait que la formulation bactérienne séchée peut être conservée à des températures proches de l'ambiante (environ 20°C) et les bactéries peuvent retrouver leur activité lors de leur réhydratation en milieux aqueux.

Un autre aspect de l'invention concerne les formulations bactériennes séchées, obtenues ou susceptibles d'être obtenues par le procédé susmentionné.

L'invention concerne également l'utilisation desdites formulations bactériennes dans des compositions des domaines aussi divers que la cosmétique, l'alimentaire, la détergence, pharmaceutique, les matériaux de construction, les fluides de forage. Enfin, un autre aspect de la présente invention concerne les compositions cosmétiques, alimentaires, détergentes, pharmaceutiques, destinées aux matériaux de construction, aux fluides de forage, à base de formulations bactériennes séchées telles que définies plus haut.

Des exemples concrets mais non limitatifs de l'invention vont maintenant être présentés.

### EXEMPLES

### Conditions opératoires générales

### 1/ Bactéries testées

4 souches de bactéries lactiques ont été testées.

Il est utile de rappeler que les souches mésophiles se développent préférentiellement entre 20 et 40°C et les souches thermophiles entre 40 et 60°C. Il faut noter que la plupart des bactéries sont détruites au dessus de 50°C à l'exception de certaines souches thermophiles. Nous avons sélectionné des souches qui résistent plus ou moins au séchage par lyophilisation. Les souches suivantes ont été retenues :
- une souche mésophile très résistante (SL 1) - *Streptococcus lactis (Lactococcus lactis)*
- une souche mésophile moins résistante (SL 2) - *Streptococcus lactis (Lactococcus lactis)*

### 2/ Mesure du taux de survie des bactéries

Le taux de survie des bactéries est mesuré par 2 méthodes distinctes qui dépendent du type d'application considéré. Le taux de survie est la caractéristique la plus critique de la qualité du produit.

### ■ Méthode de numération

Des formulations bactériennes sont remises en suspension puis diluées puis mises en culture sur des milieux nutritifs pendant 24 heures. Le comptage des colonies de bactéries à l'issue de ces 24 heures permet, connaissant la dilution, de calculer le nombre de bactéries par gramme d'échantillon. Cette méthode, très largement employée, compte toutes les bactéries même celles endommagées par le séchage qui disposent de 24 heures pour pouvoir se réparer. Les résultats sont exprimés en pourcentage de survie entre la suspension non séchée et la poudre de formulation bactérienne séchée et remise en suspension.

### ■ Méthode d'acidification

Cette méthode consiste à mesurer l'évolution du pH en fonction du temps d'un milieu nutritif ensemencé avec une formulation bactérienne. L'activité d'une formulation bactérienne est obtenue en unité par gramme (U/g) avec une précision de ± 10%. Cette méthode, qui est celle employée dans les exemples, mesure l'activité de la formulation bactérienne dès sa remise en suspension. Elle reflète plus fidèlement l'état de la formulation bactérienne à l'issue du séchage.

### 3/ Méthodes de caractérisation

### ■ humidité résiduelle sous vide

L'objectif de cette mesure est de quantifier la quantité totale d'eau résiduelle dans le produit après chaque étape de séchage. La mesure est réalisée dans une étuve sous vide sur 2 grammes de produit placés en couche mince sous un vide compris entre 1.10³ et 1,5.10⁴ Pa pendant 2 heures. Le vide est nécessaire pour ne pas dégrader thermiquement les bactéries et les additifs.

### ■ activité de l'eau

L'activité de l'eau (aw) dans le produit après chaque étape de séchage correspond au rapport entre la pression partielle de vapeur d'eau au-dessus de l'échantillon et la pression partielle de vapeur d'eau de l'eau pure à la même température. De manière simplifiée, l'activité de l'eau reflète le degré de liberté de l'eau dans le produit considéré et elle aura une incidence forte sur la stabilité et la conservation du produit au cours de son stockage. D'après la littérature, cette mesure est linéairement corrélée à la température de transition vitreuse du produit. Cette grandeur (aw) est obtenue à 20°C à l'aide d'un dispositif qui mesure un point de rosée par refroidissement. L'appareil de marque CX2T commercialisé par la société Aqualab permet de mesurer des aw comprises entre 0,03 et 1 avec une précision de ± 0,01.

### 4/ Caractérisation de la structure du produit obtenu

Les méthodes classiques de microscopie électronique sur bris et de diffraction de rayons X permettent d'apprécier l'état de cristallisation de la formulation bactérienne.

La mesure de température de transition vitreuse permet de déterminer l'état viscoélastique de la formulation bactérienne au cours du séchage. Sa valeur est déterminante pour le choix des paramètres de séchage. Elle permet également de définir les conditions optimales de stockage.

La mesure de température de transition vitreuse est effectuée à l'aide d'une méthode calorimétrique classique. L'échantillon est porté de - 60°C à + 80°C à 20°C/min tout en suivant le dégagement de chaleur lié à la transition vitreuse. La vitesse de montée en température choisie et le mode de mesure permettent d'assurer une précision sur la valeur de la température de transition vitreuse allant de ± 2 à ± 5°C suivant la nature du produit.

### Exemple 1 : Souche mésorahile (SL, 1) - Lactococcus lactis

A une suspension comprenant 130 grammes de bactéries (SL 1) par kilogrammes de suspension, on ajoute un mélange d'additifs constitué de lactose (35%), saccharose (20%), glutamate de sodium (15%), lactosérum (30%) dans un rapport bactéries/additifs de 1.

La température de la suspension ainsi obtenue est d'environ 2 à 4°C. Cette suspension est séchée d'abord dans un atomiseur de type MINOR commercialisé par la société NIRO à l'aide de la configuration turbine co-courant, et ensuite dans une étuve sous pression réduite.

La température entrée d'atomiseur choisie est de 75 ± 5°C. Deux températures différentes (33 ± 1 et 40 ± 1 °C) ont été testées en sortie d'atomiseur. La pression de l'air comprimé qui met la turbine (à 24 canaux) en rotation est comprise entre 7.10⁵ et 8.10⁵ Pa. Le gaz chaud est constitué essentiellement (concentration supérieure à 95%) d'azote.

Ces 2 exemples sont référencés exemple 1a et exemple 1b dans le tableau 1. Pour le séchage sous vide un temps de traitement de 2 heures à une température qui évolue entre 35 et 40°C sous un vide qui évolue entre 1,5.10³ et 2.10³ Pa est effectué. Les caractéristiques des produits obtenus au cours des deux étapes de séchage sont données dans le tableau 1.

Le rendement matière de l'opération est compris entre 70 et 80% pour une température de sortie de 40°C.

Les produits sortent de l'atomiseur avec une humidité résiduelle de 5,9% pour une température de sortie atomiseur de 40°C et de 7,8% pour une température sortie atomiseur de 33°C. L'humidité résiduelle est respectivement de 2,4% et de 2,7% après séchage sous vide.

Le séchage de cette formulation bactérienne par atomisation puis sous vide donne des taux de survie des bactéries comparables à ceux obtenus par lyophilisation.

La diffraction par rayons X et la microscopie électronique sur bris confirme l'obtention d'une matrice amorphe.

Le séchage ultérieur sous vide jusqu'à des (aw) inférieures à 0,1 permet d'atteindre des températures de transition vitreuse des produits supérieures à 45°C ce qui permet d'envisager le stockage des produits à température ambiante (environ 20°C).

### Exemple 2 : Souche mésophile (SL 2) - Lactococcus lactis

A une suspension comprenant 130 grammes de bactéries (SL 2) par kilogrammes de suspension, on ajoute un mélange d'additifs constitué de lactose (35%), saccharose (20%), glutamate de sodium (15%), le lactosérum (30%) dans un rapport bactéries/additifs de 1.

La température de la suspension ainsi obtenue est d'environ 2 à 4°C. Cette suspension est séchée d'abord dans un atomiseur de type MINOR commercialisé par la société NIRO à l'aide de la configuration turbine co-courant, et ensuite dans une étuve sous pression réduite.

La température entrée atomiseur choisie est de 75 ± 5°C. La température en sortie atomiseur est de 40°C. La pression de l'air comprimé qui met la turbine (à 24 canaux) en rotation est comprise entre 7.10⁵ et 8.10⁵ Pa. Le gaz chaud est constitué essentiellement (concentration supérieure à 95%) d'azote.

Pour le séchage sous vide, un temps de traitement de 2 heures à une température qui évolue entre 36 et 39°C sous un vide qui évolue entre 1,8 .10³ et 2 .10³ Pa est effectué. Les caractéristiques des produits obtenus au cours des deux étapes de séchage sont données dans le tableau 1.

Le rendement matière de l'opération est de 70%.

Les produits sortent de l'atomiseur avec une humidité résiduelle de 6,5%. L'humidité résiduelle est de 2,1% après séchage sous vide.

Le séchage de cette formulation bactérienne par atomisation puis sous vide donne des taux de survie des bactéries comparables à ceux obtenus par lyophilisation.

La diffraction par rayons X et la microscopie électronique sur bris confirme l'obtention d'une matrice amorphe.

Le séchage ultérieur sous vide jusqu'à une aw de 0,06 permet d'atteindre une température de transition vitreuse du produit de 54°C ce qui permet encore plus que dans l'exemple 1 d'envisager un stockage du produit à température ambiante. Ce séchage poussé est réalisé sans dégradation de l'activité bactérienne par rapport à la lyophilisation.

**Tableau 1**

| | exemple 1a | exemple 1b | exemple 2 |
|---|---|---|---|
| température entrée A (°C) | 75 | 75 | 75 |
| température sortie A (°C) | 33 | 40 | 40 |
| température SS V (°C) | 35-40 | 35-40 | 36-39 |
| H.R. sortie A (%) | 7,8 | 5,9 | 6,5 |
| aw sortie A (%) | 0,36 | 0,30 | 0,33 |
| H.R. sortie SS V (%) | 2,4 | 2,7 | 2,1 |
| aw sortie SS V (%) | 0,07 | 0,09 | 0,06 |
| Tg sortie A (°C) | 7 | 14 | 10 |
| Tg sortie SS V (°C) | 52 | 48 | 54 |
| **% activité / lyophilisation *** | **101** | **109** | **94** |

| | | | |
|---|---|---|---|
| A : atomiseur, SS V : sous vide, H.R. : humidité résiduelle, Tg : température de transition vitreuse, act. : activité de la formulation bactérienne. * : % activité / lyophilisation : représente le pourcentage de taux de survie après atomisation et séchage sous vide par rapport à la lyophilisation. | | | |

### Exemple 3 : Souche thermophile sensible (LH 1) - Streptococcus thermophilus

A une suspension comprenant 120 grammes de bactéries (LH 1) par kilogrammes de suspension, on ajoute un mélange d'additifs constitué de lactose (25%), saccharose (25%), lait de vache (50%) dans un rapport bactéries/additifs de 1.

La température de la suspension ainsi obtenue est d'environ 2 à 4°C. Cette suspension est séchée d'abord dans un atomiseur de type MINOR commercialisé par la société NIRO à l'aide de la configuration turbine co-courant, et ensuite dans une étuve sous pression réduite.

La température entrée d'atomiseur choisie est de 75 ± 2°C. La température en sortie de l'atomiseur est de (40 ± 1°C). La pression de l'air comprimé qui met la turbine (à 24 canaux) en rotation est comprise entre 7.10⁵ et 8.10⁵ Pa. Le gaz chaud est constitué essentiellement (concentration supérieure à 95%) d'azote.

Pour le séchage sous vide un temps de traitement de 2 heures à une température de 35°C sous un vide qui évolue entre 1,5.10³ et 2.10³ Pa est effectué. Les caractéristiques des produits obtenus au cours des deux étapes de séchage sont données dans le tableau 2.

Les produits sortent de l'atomiseur avec une aw de 0,31 correspondant à une humidité résiduelle de 6,5% pour une température de sortie atomiseur de 40°C. Une aw de 0,08 correspondant à une humidité résiduelle de 2,5% après séchage sous vide.

Le séchage de cette formulation bactérienne par atomisation puis sous vide donne des taux de survie des bactéries supérieurs à ceux obtenus par lyophilisation.

La diffraction par rayons X et la microscopie électronique sur bris confirme l'obtention d'une matrice amorphe.

Le séchage ultérieur sous vide jusqu'à des (aw) inférieures à 0,1 permet d'atteindre des températures de transition vitreuse des produits supérieures à 33°C ce qui permet d'envisager le stockage des produits à température ambiante (environ 20°C).

### Exemple 4 : Souche thermophile résistante (ST 1) - Streptococcus thermophilus

A une suspension comprenant 130 grammes de bactéries (ST 1) par kilogrammes de suspension, on ajoute un mélange d'additifs constitué de lactose (35%), saccharose (20%), glutamate de sodium (15%), le lactosérum (30%) dans un rapport bactéries/additifs de 1.

La température de la suspension ainsi obtenue est d'environ 2 à 4°C. Cette suspension est séchée d'abord dans un atomiseur de type MINOR commercialisé par la société NIRO à l'aide de la configuration turbine co-courant, et ensuite dans une étuve sous pression réduite.

La température entrée atomiseur choisie est de 75 ± 2°C. La température en sortie atomiseur est de 40°C. La pression de l'air comprimé qui met la turbine (à 24 canaux) en rotation est comprise entre 7.10⁵ et 8.10⁵ Pa. Le gaz chaud est constitué d'air.

Pour le séchage sous vide, un temps de traitement de 5 heures à une température qui évolue entre 36 et 39°C sous un vide qui évolue entre 1,5 .10³ et 2 .10³ Pa est effectué. Les caractéristiques des produits obtenus au cours des deux étapes de séchage sont données dans le tableau 2.

Les produits sortent de l'atomiseur avec une aw de 0,3 correspondant à une humidité résiduelle de 6,0%. Une aw de 0,1 correspondant à une humidité résiduelle de 2,8% après séchage sous vide.

Le séchage par atomisation puis sous vide donne des taux de survie des bactéries comparables à ceux obtenus par lyophilisation.

Le séchage ultérieur sous vide jusqu'à une aw de 0,1 permet d'atteindre une température de transition vitreuse du produit de 42°C ce qui permet encore plus que comme dns les exempls précédents d'envisager un stockage du produit à température ambiante. Ce séchage poussé est réalisé sans dégradation de l'activité bactérienne par rapport à la lyophilisation.

**Tableau 2**

| | exemple 3 | exemple 4 |
|---|---|---|
| température entrée A (°C) | 75 | 75 |
| température sortie A (°C) | 40 | 40 |
| température SS V (°C) | 35 | 36-39 |
| H.R. sortie A (%) | 6,5 | 6,0 |
| aw sortie A (%) | 0,31 | 0,3 |
| H.R. sortie SS V (%) | 2,5 | 2,8 |
| aw sortie SS V (%) | 0,08 | 0,1 |
| Tg sortie A (°C) | 8,5 | 12 |
| Tg sortie SS V (°C) | 33 | 42 |
| **% activité / lyophilisation *** | **138** | **74** |

| | | |
|---|---|---|
| A : atomiseur, SS V : sous vide, H.R. : humidité résiduelle, Tg : température de transition vitreuse, act. : activité de la formulation bactérienne. % activité / lyophilisation : représente le pourcentage de taux de survie après atomisation et séchage sous vide par rapport à la lyophilisation. | | |

## Revendications

1. Procédé de séchage de bactéries **caractérisé en ce que :**
*a)* l'on pulvérise une suspension aqueuse d'au moins une bactérie représentant au moins 3% de la matière sèche de la suspension, dans une atmosphère chaude (atomisation) dont la température est d'au plus 500°C pendant un temps adapté pour obtenir une poudre dont la teneur résiduelle en eau est d'au moins 5 % en poids par rapport au poids total de la poudre, et
*b)* l'on soumet la poudre issue de l'étape *(a)* à une étape de séchage sous pression réduite d'au plus 3.10⁴ Pa pour obtenir une poudre dont la teneur résiduelle en eau est d'au plus 4 % en poids par rapport au poids total de la poudre.

2. Procédé selon la revendication 1, **caractérisé en ce que** la poudre issue de l'étape *(a)* possède une teneur résiduelle en eau comprise entre 5 et 30% en poids, et préférentiellement entre 7 et 20 % en poids par rapport au poids total de la poudre

3. Procédé selon l'une des revendications 1 ou 2, **caractérisé en ce que** l'atomisation est effectuée à une température des gaz chauds à l'entrée d'au plus 500°C, avantageusement entre 70 et 300°C et plus préférentiellement entre 70°C et 150°C.

4. Procédé selon l'une quelconque des revendications 1 à 3, **caractérisé en ce qu'**à l'issue de l'étape *(a)* la poudre a une granulométrie comprise entre 1 et 100 microns, et préférentiellement entre 5 et 50 microns.

5. Procédé selon l'une quelconque des revendications 1 à 4**, caractérisé en ce que** le séchage dans l'étape *(b)* a lieu sous une pression réduite comprise entre 1.10³ et 1,5.10⁴ Pa, de préférence entre 1,5.10³ et 1.10⁴ Pa

6. Procédé selon l'une quelconque des revendications 1 à 5, **caractérisé en ce que** le séchage dans l'étape *(b)* a lieu à une température comprise entre 20 et 60°C, de préférence entre 30 et 50°C.

7. Procédé selon l'une quelconque des revendications 1 à 6, **caractérisé en ce que** la poudre issue de l'étape *(b)* possède une teneur résiduelle en eau comprise entre 1 et 4% en poids, et préférentiellement entre 1 et 3 % en poids par rapport au poids total de la poudre.

## Claims

1. A process for drying bacteria,
**characterized in that:**
a) an aqueous suspension of at least one bacterium representing at least 3% of the dry matter of the suspension, is sprayed into a hot atmosphere (atomization), the temperature of which is at most 500°C for a time suitable for obtaining a powder whose residual water content is at least 5% by weight with respect to the total weight of the powder; and
b) the powder resulting from step (a) is subjected to a step of drying under reduced pressure of at most 3 x 10⁴ Pa in order to obtain a powder whose residual water content is at most 4% by weight with respect to the total weight of the powder.

2. The process as claimed in claim 1, **characterized in that** the powder resulting from step (a) possesses a residual water content of between 5 and 30% by weight, and preferably between 7 and 20% by weight, with respect to the total weight of the powder.

3. The process as claimed in either of claims 1 and 2, **characterized in that** the atomization is carried out at a hot-gas inlet temperature of at most 500°C, advantageously between 70°C and 300°C and more preferably between 70°C and 150°C.

4. The process as claimed in any one of claims 1 to 3, **characterized in that** after step (a) the powder has a particle size of between 1 and 100 microns and preferably between 5 and 50 microns.

5. The process as claimed in any one of claims 1 to 4, **characterized in that** the drying in step (b) takes place under a reduced pressure of between 1 x 10³ and 1.5 x 10⁴ Pa, preferably between 1.5 x 10³ and 1 x 10⁴ Pa.

6. The process as claimed in any one of claims 1 to 5, **characterized in that** the drying in step (b) takes place at a temperature of between 20 and 60°C, preferably between 30 and 50°C.

7. The process as claimed in any one of claims 1 to 6, **characterized in that** the powder resulting from step (b) possesses a residual water content of between 1 and 4% by weight, and preferably between 1 and 3% by weight, with respect to the total weight of the powder.

## Patentansprüche

1. Verfahren zur Trocknung von Bakterien, **dadurch gekennzeichnet, dass** man:
a) eine wässrige Suspension wenigstens eines Bakteriums, welches wenigstens 3 % der Trockenmasse der Suspension ausmacht, in heißer Atmosphäre, deren Temperatur höchstens 500°C beträgt, über einer Zeitraum, der derart eingestellt ist, dass ein Pulver erhalten wird, dessen Restgehalt an Wasser, bezogen auf das Gesamtgewicht des Pulvers, wenigstens 5 Gew.- % beträgt, pulverisiert (Zerstäubung),
b) das aus Schritt (a) hervorgegangene Pulver einem Trocknungsschritt unter einem reduziertem Druck von höchstens 3 · 10⁴ Pa unterwirft, um ein Pulver zu erhalten, dessen Restgehalt an Wasser, bezogen auf das Gesamtgewicht des Pulvers, höchstens 4 Gew.- % beträgt.

2. Verfahren nach Anspruch 1, **dadurch gekennzeichnet, dass** das aus Schritt (a) hervorgehende Pulver einen Restgehalt an Wasser von 5 bis 30 Gew.-%, bevorzugt von 7 bis 20 Gew.-%, bezogen auf das Gesamtgewicht des Pulvers, aufweist.

3. Verfahren nach einem der Ansprüche 1 oder 2, **dadurch gekennzeichnet, dass** die Zerstäubung bei einer Temperatur der Heißgase am Eingang von höchstens 500 °C, vorteilhafterweise von 70 bis 300 °C und am bevorzugtesten von 70 °C bis 150 °C bewirkt wird.

4. Verfahren nach irgendeinem der Ansprüche 1 bis 3, **dadurch gekennzeichnet, dass** am Ende des Schritts (a) das Pulver eine Korngröße (Granulometrie) von 1 bis 100 Mikrometern, bevorzugt von 5 bis 50 Mikrometern, aufweist.

5. Verfahren nach irgendeinem der Ansprüche 1 bis 4, **dadurch gekennzeichnet, dass** die Trocknung in Schritt (b) unter einem reduzierten Druck von 1 · 10³ bis 1,5 · 10⁴ Pa, bevorzugt von 1,5 · 10³ bis 1 · 10⁴ Pa, stattfindet.

6. Verfahren nach irgendeinem der Ansprüche 1 bis 5, **dadurch gekennzeichnet, dass** die Trocknung in Schritt (b) bei einer Temperatur von 20 bis 60 °C, bevorzugt von 30 bis 50 °C, stattfindet

7. Verfahren nach irgendeinem der Ansprüche 1 bis 6, **dadurch gekennzeichnet, dass** das aus Schritt (b) hervorgehende Pulver einen Restgehalt an Wasser von 1 bis 4 Gew.-%, bevorzugt von 1 bis 3 Gew.-%, bezogen auf das Gesamtgewicht des Pulvers, aufweist.
